# FASCICULE DE BREVET EUROPEEN

(11) **EP 1 140 801 B1**
(45) Date de publication et mention de la délivrance du brevet: **23.02.2005**
(21) Numéro de dépôt: 99959509.3
(22) Date de dépôt: 21.12.1999
(51) Int. Cl.: C07C 253/10

(54) **PROCEDE D'HYDROCYANATION DE COMPOSES ORGANIQUES A INSATURATIONS ETHYLENIQUES**
VERFAHREN ZUR HYDROCYANIERUNG VON ORGANISCHEN ETHYLENISCH UNGESÄTTIGTEN VERBINDUNGEN
HYDROCYANATION METHOD FOR ETHYLENICALLY UNSATURATED ORGANIC COMPOUNDS

(30) Priorité: 22.12.1998 FR 9816468
(43) Date de publication de la demande: 10.10.2001
(73) Titulaire: Rhodia Polyamide Intermediates, 69192 Saint-Fons (FR)
(72) Inventeur: BURATTIN, Paolo, F-69002 Lyon (FR); COQUERET, Pierre, F-69340 Francheville (FR); HUSER, Marc, F-69100 Villeurbanne (FR)
(74) Mandataire: Esson, Jean-Pierre
(86) Numéro de dépôt international: PCT/FR1999/003231
(87) Numéro de publication internationale: WO 2000/037431

(56) Documents cités:
- EP-A- 0 160 296
- EP-A- 0 650 959
- WO-A-97/12857
- DE-A- 2 807 849
- US-A- 4 990 645

## Description

La présente invention concerne un procédé d'hydrocyanation de composés organiques à insaturations éthyléniques pour notamment l'obtention de nitriles, et plus particulièrement l'hydrocyanation de dioléfines ou d'oléfines substituées pour la fabrication de dinitriles, et/ou l'isomérisation des nitriles obtenus par hydrocyanation.

L'invention se rapporte plus particulièrement à une hydrocyanation catalysée par un composé à base de nickel.

Le brevet français n° 1 599 761 décrit un procédé de préparation de nitriles par addition d'acide cyanhydrique sur des composés organiques ayant au moins une double liaison éthylénique, en présence d'un catalyseur au nickel et d'une triarylphosphite. Cette réaction peut être conduite en présence ou non d'un solvant.

Lorsqu'un solvant est utilisé dans ce procédé de l'art antérieur, il s'agit de préférence d'un hydrocarbure, tel que le benzène ou les xylènes ou d'un nitrile tel que l'acétonitrile.

Le catalyseur mis en oeuvre est un complexe organique de nickel, contenant des ligands tels que les phosphines, les arsines, les stilbines, les phosphites, les arsénites ou les antimonites.

La présence d'un promoteur pour activer le catalyseur, tel qu'un composé du bore ou un sel métallique, généralement un acide de Lewis, est également préconisée dans ledit brevet.

Le brevet FR-A-2 338 253 a proposé de réaliser l'hydrocyanation des composés ayant au moins une insaturation éthylénique, en présence d'une solution aqueuse d'un composé d'un métal de transition, notamment le nickel, le palladium ou le fer, et d'une phosphine sulfonée.

Les phosphines sulfonées décrites dans ce brevet sont des triarylphosphines sulfonées et plus particulièrement des triphénylphosphines sulfonées.

Ce procédé permet une bonne hydrocyanation, notamment du butadiène et des pentène-nitriles, une séparation aisée de la solution catalytique par simple décantation et par conséquent évite au maximum le rejet d'effluents ou de déchets contenant les métaux servant de catalyseur.

Toutefois, dans ces procédés la durée de vie totale du catalyseur est affectée par une perte en nickel lors de chaque passage du catalyseur dans le réacteur. Cette perte est due notamment à un précipité d'hydroxyde de nickel. Ce précipité présente également un inconvénient dans le fonctionnement du procédé car il pollue les installations requérant soit un nettoyage périodique de celles-ci soit une étape de séparation et élimination du précipité. Dans ce dernier mode de réalisation, il y a production d'un effluent gênant pour l'environnement.
Il a été proposé un procédé de recyclage d'un catalyseur d'hydrocyanation d'oléfine en solution organique par le brevet DE 1 0807 849. Ce procédé consiste à récupérer le précipité de catalyseur à base de nickel par la dissolution par une solution d'ammoniaque pour éliminer le précipité de nickel avant recyclage.

Un des objets de la présente invention est de remédier à ces inconvénients en proposant un procédé permettant de traiter le précipité pour d'une part supprimer les risques de pollution des installations ou de l'environnement et d'autre part diminuer les pertes en catalyseur.

A cet effet, l'invention propose un procédé d'hydrocyanation de composés organiques comprenant au moins une liaison éthylénique par réaction avec le cyanure d'hydrogène, en présence d'une solution aqueuse d'un catalyseur comprenant au moins un composé de nickel et au moins une phosphine hydrosoluble, et séparation après réaction des phases organique et aqueuse.

Le procédé de l'invention consiste à traiter la phase aqueuse récupérée, qui comprend une phase solide (colloïdes, gel ou solide en suspension ou précipité) formée au cours de la réaction d'hydrocyanation, par du cyanure d'hydrogène pour dissoudre ladite phase solide.

Ainsi, la phase aqueuse peut être recyclée au moins partiellement dans le réacteur sans provoquer de pollution de l'installation.

Dans un autre mode de réalisation de l'invention, la phase aqueuse A récupérée contenant la phase solide formée est soumise à une étape de séparation pour extraire la phase solide, la phase aqueuse A₁ épurée ou clarifiée étant ensuite au moins partiellement recyclée dans le réacteur d'hydrocyanation, la phase solide étant solubilisée par traitement avec du cyanure d'hydrogène.

Selon une caractéristique préférentielle de l'invention, le traitement de la phase solide séparée par du cyanure d'hydrogène est réalisé en présence d'une solution aqueuse contenant une phosphine hydrosoluble. La solution **B** ainsi récupérée est, dans un mode de réalisation préféré alimentée dans une étape de régénération du catalyseur consistant brièvement en une réduction du nickel à l'état d'oxydation (II) en du nickel à l'état d'oxydation (0). Cette réduction est mise en oeuvre, avantageusement après addition de nickel à l'état d'oxydation (0) dans la solution obtenue par dissolution de la phase solide.

Selon une caractéristique préférée de l'invention, la solution B est mélangée avec au moins une partie de la solution aqueuse A₁ clarifiée avant le traitement de régénération par réduction. Ainsi, comme cette solution A₁ contient du nickel à l'état d'oxydation (0), la régénération du catalyseur dans sa totalité est possible.

Selon une caractéristique de l'invention, le traitement par le cyanure d'hydrogène de la phase solide formée, soit après clarification de la phase liquide A récupérée soit directement dans cette phase liquide est réalisée par addition de cyanure d'hydrogène dans la solution aqueuse contenant avantageusement une phosphine hydrosoluble ou catalyseur d'hydrocyanation, et maintient sous agitation du milieu réactionnel pendant le temps nécessaire à la dissolution de préférence complète du précipité.
La température du milieu réactionnel est avantageusement inférieure à 100°C, préférentiellement comprise entre 20°C et 80°C. Le cyanure d'hydrogène est ajouté sous forme liquide ou en solution dans de l'eau ou dans une solution de phosphine hydrosoluble.

La quantité de cyanure d'hydrogène ajoutée est au moins égale à la quantité stoechiométrique pour transformer le nickel précipité ou insoluble en cyanure de nickel soluble. Cette quantité minimale peut être déterminée en fonction du poids de précipité à dissoudre et en considérant par hypothèse que ce Ni précipité ou insoluble est de l'hydroxyde de nickel.

Avantageusement, la quantité de cyanure d'hydrogène ajoutée sera de 30 % à 400 % supérieure à la quantité stoechiométrique.

L'étape de régénération du catalyseur ou en d'autres termes la réduction du nickel à l'état d'oxydation 0 peut être réalisée par plusieurs procédés tels que la réduction par de l'hydrogène gazeux, par réduction électrochimique ou addition d'un agent réducteur organique ou minéral choisi dans le groupe comprenant les borohydrures, le magnésium, le zinc. Les procédés de réduction sont connus et décrits notamment dans les brevets WO 97/24184, EP 0 715 890 et FR2 338 253.

Les composés phosphines hydrosolubles convenables pour mettre en oeuvre l'invention peuvent être un des composés appartenant à la famille des composés phosphines décrits dans le brevet français n° 2 338 253 ou dans les demandes de brevet WO 97.12857 et EP 0 650 959.

Ainsi des phosphines convenables pour l'invention répondent à la formule générale (I) suivante : 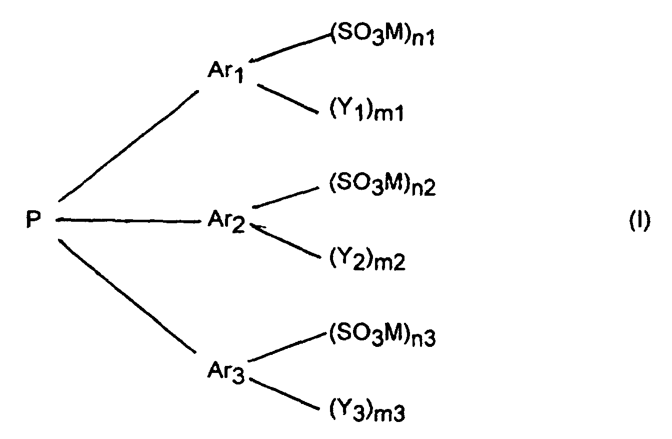 dans laquelle :
* Ar₁, Ar₂ et Ar₃, identiques ou différents, représentent des groupements aryles
* Y₁, Y₂ et Y₃, identiques ou différents, représentent
   . un radical alkyle ayant 1 à 4 atomes de carbone,
   . un radical alcoxy ayant 1 à 4 atomes de carbone,
   . un atome d'halogène,
   . un radical CN,
   . un radical NO₂
   . un radical OH,
   . un radical NR₁R₂, dans la formule duquel R₁ et R₂, identiques ou différents, représentent un radical alkyle ayant 1 à 4 atomes de carbone.
* M est un reste cationique minéral ou organique choisi, de manière à ce que le composé de formule (I) soit soluble dans l'eau, dans le groupe comprenant :
   . H⁺,
   . les cations dérivés des métaux alcalins ou alcalino-terreux,
   . N(R₃R₄R₅R₆)⁺ avec R₃,R₄,R₅ et R₆, identiques ou différents, représentant un radical alkyle ayant 1 à 4 atomes de carbone ou un atome d'hydrogène,
   . les autres cations dérivés des métaux dont les sels de l'acide benzènesulfonique sont solubles dans l'eau,
* m₁, m₂ et m₃ sont des nombres entiers, identiques ou différents, de 0 à 5,
* n₁, n₂ et n₃ sont des nombres entiers, identiques ou différents, de 0 à 3, l'un d'entre eux au moins étant égal ou supérieur à 1.

Comme exemples de métaux dont les sels de l'acide benzènesulfonique sont solubles dans l'eau, on peut citer le plomb, le zinc et l'étain.

Par l'expression soluble dans l'eau, on entend de manière générale dans le présent texte un composé soluble à au moins 0,01 g par litre d'eau.

Parmi les phosphines de formule (I), on préfère celles pour lesquelles :
- Ar₁, Ar₂ et Ar₃ sont des groupements phényle,
- Y₁, Y₂ et Y₃ représentent des groupements choisis parmi
   . les radicaux alkyle ayant de 1 à 2 atomes de carbone,
   . les radicaux alkoxy ayant de 1 à 2 atomes de carbone,
- M représente un cation choisi parmi le groupe comprenant
   . H⁺
   . les cations dérivés de Na, K, Ca, Ba
   . NH₄⁺
   . les cations tétraméthylammonium, tétraéthylammonium, tétrapropylammonium, tétrabutylammonium
- m₁, m₂ et m₃ sont des nombres entiers de 0 à 3
- n₁, n₂ et n₃ sont des nombres entiers de 0 à 3, l'un au moins étant également supérieur à 1.

Parmi ces phosphines, les plus particulièrement préférées sont les sels de sodium. de potassium, de calcium, de baryum, d'ammonium, de tétraméthylammonium et de tétraéthylammonium, des mono(sulfophényl) diphényl-phosphine, di(sulfophényl) phényl-phosphine et tri(sulfophényl)-phosphine dans les formules desquelles les groupements SO₃ sont de préférence en position méta.

On peut citer comme autres exemples de phosphines de formule (I) pouvant être mises en oeuvre dans le procédé de l'invention, les sels alcalins ou alcalino-terreux, les sels d'ammonium, les sels d'ammonium quaternaire des (sulfo-3 méthyl-4 phényl) di (méthyl-4 phényl)-phosphine ; (sulfo-3 méthoxy-4 phényl) di(méthoxy-4 phényl)-phosphine ; (sulfo-3 chloro-4 phényl) di(chloro-4 phényl)-phosphine ; di(sulfo-3 phényl) phényl-phosphine ; di(sulfo-4 phényl) phényl-phosphine ; di(sulfo-3 méthyl-4 phényl) (méthyl-4 phényl)-phosphine ; di(sulfo-3 méthoxy-4 phényl) (méthoxy-4 phényl) phosphine ; di(sulfo-3 chloro-4 phényl) (chloro-4 phényl) phosphine ; tri(sulfo-3 phényl)-phosphine ; tri(sulfo-4 phényl)-phosphine ; (tri(sulfo-3 méthyl-4 phényl)-phosphine ; tri(sulfo-3, méthoxy-4 phényl)-phosphine ; tri(sulfo-3 chloro-4 phényl)-phosphine ; (sulfo-2 méthyl-4 phényl) (sulfo-3, méthyl-4 phényl) (disulfo-3,5 méthyl-4 phényl)-phosphine ; (sulfo-3 phényl) (sulfo-3 chloro-4 phényl) (disulfo-3,5 chloro-4 phényl)-phosphine.

On peut bien évidemment utiliser un mélange de ces phosphines. En particulier, on peut utiliser un mélange de phosphines mono-, di- et tri-métasulfonées. Des phosphines monodentates ou bidentates de formules générales (II) et (III) suivantes sont également convenables pour l'invention : 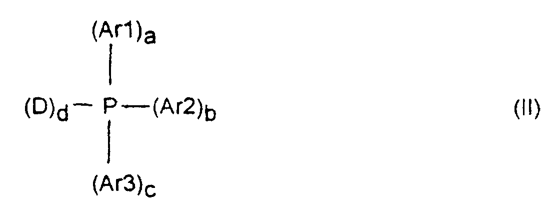 dans laquelle :
- Ar1 et Ar2, identiques ou différents, représentent des groupes aryle ou aryle comportant un ou plusieurs substituants tels que :
   - radical alkyle ou alcoxy ayant 1 à 4 atomes de carbone,
   - atome d'halogène,
   - groupe hydrophile comme :
      -COOM, -SO₃M, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides arylphosphoniques sont solubles dans l'eau,
      -N(R)₄ dans la formule duquel les symboles R, identiques ou différents, représentent un atome d'hydrogène ou un radical alkyle ayant de 1 à 4 atomes de carbone,
      -OH
- Ar3 représente un groupe aryle comportant un ou plusieurs substituants tels que :
   - radical alkyle ou alcoxy ayant 1 à 4 atomes de carbone.
   - atome d'halogène,
   - groupe hydrophile comme :
      -COOM, -PO₃M, M représentant un reste cationique minéral ou organique choisi parmi le proton, les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques ou des acides arylphosphoniques sont solubles dans l'eau,
      -N(R)₄ dans la formule duquel les symboles R, identiques ou différents, représentent un atome d'hydrogène ou un radical alkyle ayant de 1 à 4 atomes de carbone,
      -OH,
l'un au moins desdits substituants de Ar3 étant un groupe hydrophile tel que défini précédemment,
- a représente 0 ou 1,
- b représente 0 ou 1,
- c représente un nombre entier de 0 à 3,
- D représente un groupe alkyle, un groupe cycloalkyle, un groupe alkyle ou cycloalkyle comportant un ou plusieurs substituants tels que :
   - radical alcoxy ayant 1 à 4 atomes de carbone,
   - atome d'halogène,
   - groupe hydrophile comme :
      -COOM. -SO₃M, -PO₃M. M représentant un reste cationique minéral ou organique choisi parmi le proton. les cations dérivés des métaux alcalins ou alcalino-terreux, les cations ammonium -N(R)₄ dans la formule desquels les symboles R, identiques ou différents, représentent un atome d'hydrogène ou un radical alkyle ayant de 1 à 4 atomes de carbone, les autres cations dérivés de métaux dont les sels des acides arylcarboxyliques, des acides arylsulfoniques ou des acides phosphoniques sont solubles dans l'eau,
      -N(R)₄ dans la formule duquel les symboles R, identiques ou différents, représentent un atome d'hydrogène ou un radical alkyle ayant de 1 à 4 atomes de carbone,
      -OH
- d représente un nombre entier de 0 à 3,
- la somme (a+b+c+d) est égale à 3. 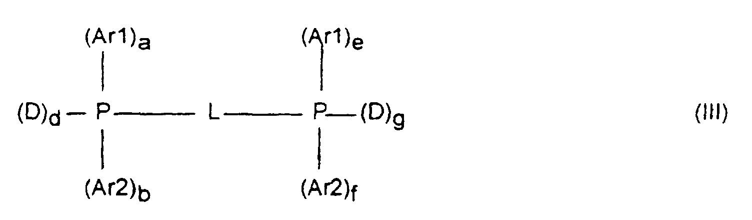
dans laquelle :
- Ar1, Ar2 et D ont les significations indiquées précédemment pour la formule (II),
- a, b, e et f représentent chacun 0 ou 1,
- d et g représentent chacun un nombre entier de 0 à 2,
- la somme (a+b+d) est égale à 2,
- la somme (e+f+g) est égale à 2,
- L représente un lien valentiel simple ou un radical hydrocarboné divalent tel qu'un radical alkylène, un radical cycloalkylène, un radical arylène, un radical dérivant d'un hétérocycle comportant un ou deux atomes d'oxygène, d'azote ou de soufre dans le cycle, ces différents radicaux cycliques étant liés directement à l'un des atomes de phosphore ou aux deux atomes de. phosphore ou étant lié à l'un des atomes de phosphore ou aux deux par l'intermédiaire d'un radical alkylène linéaire ou ramifié ayant de 1 à 4 atomes de carbone, le ou les cycles éventuellement parties du radical divalent L pouvant comporter un ou plusieurs substituants tels que groupe alkyle ayant 1 à 4 atomes de carbone.

A titre d'exemples non limitatifs de phosphines de formule générale (II), on peut citer notamment la tris(hydroxyméthyl) phosphine, la tris(2-hydroxyéthyl) phosphine, la tris(3-hydroxypropyl) phosphine, la tris(2-carboxyméthyl) phosphine. le sel de sodium de la tris(3-carboxylatophényl) phosphine, la tris(3-carboxyéthyl) phosphine, l'iodure de la tris(4-triméthylammoniumphényl) phosphine, le sel de sodium de la tris(2-phosphonatoéthyl) phosphine, la bis(2-carboxyéthyl) phényl phosphine.

A titre d'exemples non limitatifs de phosphines de formule générale (III), on peut citer notamment le sel de sodium du 2,2'-bis[di(sulfonatophényl)phosphino]-1,1'-binaphtyl, le sel de sodium du 1,2-bis[di(sulfonatophényl)phosphinométhyl] cyclobutane (CBDTS), le 1,2-bis(dihydroxyméthylphosphino) éthane, le 1,3-bis(dihydroxyméthylphosphino) propane, le sel de sodium du 2,2'-bis[di(sulfonatophényl)phosphinométhyl]-1,1'-binaphtyl.

Certaines des phosphines hydrosolubles de formule (I) à (III) sont disponibles dans le commerce.

Pour la préparation des autres, on peut se référer aux procédés généraux ou particuliers de synthèse des phosphines décrits dans les ouvrages généraux comme HOUBEN-WEYL, Method der organischen Chemie, organische Phosphor Verbindungen, teil 1 (1963).

Enfin, pour la préparation des dérivés hydrosolubles non décrits, on peut à partir des phosphines ne comportant pas de substituants hydrosolubles définis précédemment, procéder à l'introduction d'un ou plusieurs de ces substituants hydrophiles. Ainsi les groupements sulfonates par exemple peuvent être introduits par la réaction de SO₃ dans l'acide sulfurique. Les groupes carboxylates, phosphonates, ammonium quaternaires peuvent de même être introduits en appliquant les méthodes chimiques connues pour ce type de synthèse.

Les composés organiques comportant au moins une liaison éthylénique plus particulièrement mis en oeuvre dans le présent procédé sont les dioléfines comme le butadiène, l'isoprène, l'hexadiène-1,5, le cyclooctadiène-1,5, les nitriles aliphatiques à insaturation éthylénique, particulièrement les pentène-nitriles linéaires comme le pentène-3 nitrile, le pentène-4 nitrile, les monooléfines comme le styrène, le méthyl-styrène, le vinyl-naphtalène, le cyclohexène, le méthyl-cyclohexène ainsi que les mélanges de plusieurs de ces composés.

Les pentène-nitriles notamment peuvent contenir des quantités, généralement minoritaires, d'autres composés, comme le méthyl-2 butène-3 nitrile, le méthyl-2 butène-2 nitrile, le pentène-2 nitrile, le valéronitrile, l'adiponitrile, le méthyl-2 glutaronitrile, l'éthyl-2 succinonitrile ou le butadiène, provenant par exemple de la réaction antérieure d'hydrocyanation du butadiène.

Lors de l'hydrocyanation du butadiène, il se forme avec les pentène-nitriles linéaires des quantités non négligeables de méthyl-2 butène-3 nitrile et de méthyl-2 butène-2 nitrile qui pourront être isomérisés en nitriles linéaires.

La solution catalytique utilisée pour l'hydrocyanation selon le procédé de l'invention peut être préparée avant son introduction dans la zone de réaction, par exemple par addition à la solution aqueuse de la phosphine hydrosoluble de formule (I) à (III), de la quantité appropriée de composé du métal de transition choisi et éventuellement du réducteur. Il est également possible de préparer la solution catalytique "in situ" par simple mélange de ces divers constituants.

La quantité de composé du nickel utilisée est choisie de telle sorte qu'il y ait par litre de solution réactionnelle entre 10⁻⁴ et 1, et de préférence entre 0,005 et 0,5 mole de nickel.

La quantité de phosphine hydrosoluble de formule (I) à (III) utilisée pour préparer la solution réactionnelle est choisie de telle sorte que le nombre de moles de ce composé rapporté à 1 mole de nickel soit de 0,5 à 2000 et de préférence de 2 à 300.

Parmi les composés de nickel convenables, on peut citer à titre d'exemples non limitatifs :
- les composés dans lesquels le nickel est au degré d'oxydation zéro comme le tétracyanonickelate de potassium K₄ [Ni(CN)₄], le bis (acrylonitrile) nickel zéro, le bis (cyclooctadiène-1,5)₂ nickel et les dérivés contenant des ligands du groupe Va comme le tétrakis (triphényl-phosphine) nickel zéro (dans ce demier cas le composé peut être dissous dans un solvant non miscible à l'eau comme le toluène, puis une solution aqueuse de phosphine sulfonée extrait une partie du nickel en développant une coloration rouge dans la solution aqueuse qui décante);
- les composés du nickel comme les carboxylates (notamment l'acétate), carbonate, bicarbonate, borate, bromure, chlorure, citrate, thiocyanate, cyanure, formiate, hydroxyde, hydrophosphite, phosphite, phosphate et dérivés, iodure, nitrate, sulfate, sulfite, aryl- et alkyl-sulfonates.

Il n'est pas nécessaire que le composé du nickel soit lui-même soluble dans l'eau. Par exemple, le cyanure de nickel, un des composés préférés, peu soluble dans l'eau, peut être dissout dans une solution aqueuse de phosphine.

Quand le composé de nickel utilisé correspond à un état d'oxydation du nickel supérieur à 0, le catalyseur est soumis à une étape de réduction chimique ou électrochimique comme indiqué précédemment.

Bien que la réaction soit conduite généralement sans tiers solvant, il peut être avantageux de rajouter un solvant organique inerte, non miscible à l'eau, qui pourra être celui de l'extraction ultérieure.

A titre d'exemples de tels solvants, on peut citer les hydrocarbures aromatiques, aliphatiques ou cycloaliphatiques qui maintiennent à l'état biphasique le milieu réactionnel.

Ainsi dès la fin de la réaction, il est très aisé de séparer, d'une part, une phase aqueuse contenant des phosphines hydrosolubles et le composé de nickel et d'autre part, une phase organique constituée des réactifs engagés dans la réaction des produits de la réaction et le cas échéant du solvant organique non miscible à l'eau.

La réaction d'hydrocyanation est généralement réalisée à une température de 10°C à 200°C et de préférence de 30°C à 120°C.

Le procédé de l'invention peut être mis en oeuvre de manière continue ou discontinue.

Le cyanure d'hydrogène mis en oeuvre peut être préparé à partir des cyanures métalliques, notamment le cyanure de sodium, ou des cyanhydrines.

Le cyanure d'hydrogène est introduit dans le réacteur sous forme gazeuse ou sous forme liquide. Il peut également être préalablement dissous dans un solvant organique.

Dans le cadre d'une mise en oeuvre discontinue, on peut en pratique charger dans un réacteur, préalablement purgé à l'aide d'un gaz inerte (tel qu'azote, argon), soit une solution aqueuse contenant la totalité ou une partie des divers constituants tels que la phosphine hydrosoluble, le composé de métal de transition, les éventuels réducteur et solvant, soit séparément lesdits constituants. Généralement le réacteur est alors porté à la température choisie, puis le composé à hydrocyaner est introduit. Le cyanure d'hydrogène est alors lui-même introduit, de préférence de manière continue et régulière.

Lorsque la réaction (dont on peut suivre l'évolution par dosage de prélèvements) est terminée, le mélange réactionnel est soutiré après refroidissement et les produits de la réaction sont isolés par décantation, éventuellement suivie d'une extraction de la couche aqueuse à l'aide d'un solvant approprié, tel que par exemple les solvants non miscibles à l'eau cités précédemment.

La solution catalytique aqueuse peut alors être recyclée dans une nouvelle réaction d'hydrocyanation de composés organiques comportant au moins une double liaison éthylénique, après avoir été traitée conformément au procédé de l'invention.

Il est également possible d'utiliser le catalyseur en association avec un acide de Lewis.

L'acide de Lewis utilisé comme cocatalyseur permet notamment, dans le cas de l'hydrocyanation des nitriles aliphatiques à insaturation éthylénique, d'améliorer la linéarité des dinitriles obtenus, c'est-à-dire le pourcentage de dinitrile linéaire par rapport à la totalité des dinitriles formés, et/ou d'augmenter la durée de vie du catalyseur.

Par acide de Lewis, on entend dans le présent texte, selon la définition usuelle, des composés accepteurs de doublets électroniques.

On peut mettre en oeuvre notamment les acides de Lewis cités dans l'ouvrage édité par G.A. OLAH "Friedel-Crafts and related Reactions", tome I, pages 191 à 197 (1963).

Les acides de Lewis qui peuvent être mis en oeuvre comme cocatalyseurs dans le présent procédé sont choisis parmi les composés des éléments des groupes Ib, IIb, IIIa, IIIb, IVa, IVb, Va, Vb, VIb, VIIb et VIII de la Classification périodique des éléments, dans la mesure où lesdits composés sont au moins partiellement solubles dans l'eau. Ces composés sont le plus souvent des sels, notamment des halogénures, de préférence chlorures et bromures, des sulfates, des carboxylates et des phosphates.

A titre d'exemples non limitatifs de tels acides de Lewis, on peut citer le chlorure de zinc, le bromure de zinc, l'iodure de zinc, le chlorure de manganèse, le bromure de manganèse, le chlorure de cadmium, le bromure de cadmium, le chlorure stanneux. le bromure stanneux, le sulfate stanneux, le tartrate stanneux, les chlorures ou bromures des éléments des terres rares comme le lanthane, le cérium, le praséodyme. le néodyme, le samarium, l'europium, le gadolinium, le terbium, le dysprosium. l'holmium, l'erbium, le thulium, l'ytterbium et le lutétium, le chlorure de cobalt, le chlorure ferreux, le chlorure d'yttrium.

On peut bien entendu mettre en oeuvre des mélanges de plusieurs acides de Lewis.

Il est également intéressant le cas échéant de stabiliser l'acide de Lewis en solution aqueuse par l'adjonction d'un halogénure de métal alcalin ou alcalino-terreux comme le chlorure de lithium, le chlorure de sodium, le chlorure de calcium ou le chlorure de magnésium notamment. Le rapport molaire halogénure de métal alcalin ou alcalino-terreux/acide de Lewis varie de manière très large, par exemple de 0 à 100 le rapport particulier pouvant être ajusté selon la stabilité dans l'eau de l'acide de Lewis.

Parmi les acides de Lewis, on préfére tout particulièrement le chlorure de zinc, le bromure de zinc, le chlorure stanneux, le bromure stanneux, le chlorure stanneux stabilisé par le chlorure de lithium, le chlorure stanneux stabilisé par le chlorure de sodium, les mélanges chlorure de zinc/chlorure stanneux.

Le cocatalyseur acide de Lewis mis en oeuvre représente généralement de 0,01 à 50 moles par mole de composé de nickel, et de préférence de 1 à 10 mole par mole.

Comme pour la mise en oeuvre du procédé de base de l'invention, la solution catalytique utilisée pour l'hydrocyanation en présence d'acide de Lewis peut être préparée avant son introduction dans la zone de réaction, par exemple par addition à la solution aqueuse de la phosphine hydrosoluble de formule (I) à (III), de la quantité appropriée de composé de nickel, de l'acide de Lewis et éventuellement du réducteur. Il est également possible de préparer la solution catalytique "in situ" par simple mélange de ces divers constituants.

Il est également possible dans les conditions du procédé d'hydrocyanation de la présente invention, et notamment en opérant en présence du catalyseur décrit précédemment comportant au moins une phosphine hydrosoluble de formule (I) à (III) et au moins un composé de nickel, de réaliser, en absence de cyanure d'hydrogène, l'isomérisation du méthyl-2 butène-3 nitrile en pentène-nitriles.

Le méthyl-2 butène-3 nitrile soumis à l'isomérisation selon l'invention peut être mis en oeuvre seul ou en mélange avec d'autres composés.

Ainsi on peut engager du méthyl-2 butène-3 nitrile en mélange avec du méthyl-2 butène-2 nitrile, du pentène-4 nitrile, du pentène-3 nitrile, du pentène-2 nitrile, du butadiène, de l'adiponitrile, du méthyl-2 glutaroronitrile, de l'éthyl-2 succinonitrile ou du valéronitrile.

Ainsi, il est particulièrement intéressant de traiter le mélange réactionnel provenant de l'hydrocyanation du butadiène par HCN en présence d'une solution aqueuse d'au moins une phosphine hydrosoluble d'au moins un composé de nickel, plus préférentiellement d'un composé du nickel au degré d'oxydation 0, tel que défini précédemment.

Dans le cadre de cette variante préférée, le système catalytique étant déjà présent pour la réaction d'hydrocyanation du butadiène, il suffit d'arrêter toute introduction de cyanure d'hydrogène, pour laisser se produire la réaction d'isomérisation.

On peut, le cas échéant, dans cette variante faire un léger balayage du réacteur à l'aide d'un gaz inerte comme l'azote ou l'argon par exemple, afin de chasser l'acide cyanhydrique qui pourrait être encore présent.

La réaction d'isomérisation est généralement réalisée à une température de 10°C à 200°C et de préférence de 60°C à 120°C.

Dans le cas préféré d'une isomérisation suivant immédiatement la réaction d'hydrocyanation du butadiène, il sera avantageux d'opérer à la température à laquelle l'hydrocyanation a été conduite.

Comme pour le procédé d'hydrocyanation de composés à insaturation éthylénique, la solution catalytique utilisée pour l'isomérisation peut être préparée avant son introduction dans la zone de réaction, par exemple par addition à la solution aqueuse d'une phosphine hydrosoluble, de la quantité appropriée de composé de nickel et éventuellement du réducteur. Il est également possible de préparer la solution catalytique "in situ" par simple mélange de ces divers constituants. La quantité de composé de nickel, ainsi que la quantité de phosphine hydrosoluble sont les mêmes que pour la réaction d'hydrocyanation.

Bien que la réaction d'isomérisation soit conduite généralement sans tiers-solvant. il peut être avantageux de rajouter un solvant organique inerte, non miscible à l'eau, qui pourra être celui de l'extraction ultérieure. C'est notamment le cas lorsqu'un tel solvant a été mis en oeuvre dans la réaction d'hydrocyanation du butadiène ayant servi à préparer le milieu soumis à la réaction d'isomérisation. De tels solvants peuvent être choisis parmi ceux qui ont été cités précédemment pour l'hydrocyanation.

En fin de réaction, il est très aisé de séparer le catalyseur des produits de la réaction d'isomérisation comme cela a été indiqué pour l'hydrocyanation et de le recycler le cas échéant dans l'une des réactions d'hydrocyanation décrites précédemment ou dans une nouvelle réaction d'isomérisation, après traitement de la phase aqueuse et/ou de la phase solide selon le procédé de l'invention par un cyanure d'hydrogène pour dissoudre ladite phase solide.

D'autres avantages et détails de l'invention apparaîtront plus clairement au vu des exemples donnés ci-dessous uniquement à titre indicatif.

### EXEMPLE 1

### 1) Préparation de la solution catalytique Ni/TPPTS.

Dans un ballon en verre de 1 litre, muni d'un barreau aimanté et d'un réfrigérant ascendant, on charge 500 cm³ d'une solution de 350 mmol de sel de sodium de triphénylphosphine trisulfonée (TPPTS) dans l'eau; on dégaze cette solution. On introduit ensuite, sous agitation et sous courant d'argon, 21,40 g (78 mmol) de Ni(cyclooctadiène)₂, puis 350 cm³ d'ortho-xylène préalablement dégazé.

On chauffe à 45°C pendant 15 h. Après refroidissement, le système biphasique est décanté et la phase aqueuse fortement colorée en rouge est prélevée. Sa concentration en Ni déterminée par analyse est de 134 mmol/kg.

### 2) Isomérisation du méthyl-2 butène-3 nitrile

Dans un réacteur en verre de 2 litres purgé sous argon et agité à l'aide d'une turbine, on charge :
- du méthyl-2 butène-3 nitrile (2M3BN) : 770 g (9,50 mol)
- la solution catalytique : 250 cm³ (39,5 mmol de Ni et 175,5 mmol de TPPTS)

On agite le mélange réactionnel pendant 1,5 h à 90°C. Après refroidissement et décantation, la phase organique finale est dosée par chromatographie en phase gazeuse (CPG).

On obtient les résultats suivants :

| | |
|---|---|
| Taux de transformation (TT) du 2M3BN | 95 % |
| Rendement (RT) en penténe-3 nitrile ( 3PN) par rapport au 2M3BN transformé | 91,5 % |
| Rendement (RT) en méthyl-2 butène-2 nitrile (2M2BN) par rapport au 2M3BN transformé | 8 % |

La phase aqueuse récupérée après décantation présente un léger précipité (phase solide finement divisé en suspension). Au repos, ce dernier se dépose lentement sous forme d'un voile de coloration marron qui se redisperse facilement sous agitation. Un dosage du Ni sur un prélèvement aqueux filtré au moyen d'un filtre Millipore Millex-HV ® (Hydrophilic PVDF 0.45 µm) fournit la concentration en Ni suivante : 110 mmol/kg.

### 3) Traitement de la suspension aqueuse

Dans un réacteur en verre de 150 cm³ purgé à l'argon et équipé d'une turbine auto-aspirante, on charge 35 cm³ de la suspension aqueuse évoquée précédemment (§ 2). Sous agitation (1200 tours/minute) et à température ambiante, on injecte en ciel de réacteur, avec un débit constant et sur une durée d'une heure, 0,265 ml d'acide cyanhydrique par l'intermédiaire d'une seringue thermostatée à -10°C. Après injection de l'HCN, le mélange est maintenu sous agitation pendant 1 heure à température ambiante en système fermé, puis le ciel du réacteur est balayé par un flux d'argon pendant encore environ 1 heure. Par ce traitement, on obtient une solution aqueuse homogène : pas de précipité apparent au sein de la solution au repos. Un dosage du Ni total et du Ni(II), respectivement par analyse élémentaire et analyse polarographique, sur un prélèvement filtré au moyen d'un filtre Millipore Millex-HV ® (Hydrophilic PVDF 0.45 µm) fournit les résultats suivants : [Ni] = 122 mmol/kg et [Ni(II)] = 23 mmol/kg ([Ni°] = 99 mmol/kg).

### EXEMPLE 2

Dans un réacteur en verre de 150 cm³ purgé à l'argon et équipé d'une turbine auto-aspirante, on charge 35 cm³ de la suspension aqueuse évoquée dans l'exemple 1, § 2. Sous agitation (1200 tours/minute) et à une température de 50°C, on injecte en ciel de réacteur, avec un débit constant et sur une durée d'une heure, 0,265 ml d'acide cyanhydrique par l'intermédiaire d'une seringue thermostatée à -10°C. Après injection de l'HCN et retour à température ambiante, le mélange est maintenu sous agitation pendant 1 heure en système fermé, puis le ciel du réacteur est balayé par un flux d'argon pendant encore environ 1 heure. Par ce traitement, on obtient une solution aqueuse homogène : pas de précipité apparent au sein de la solution au repos. Un dosage du Ni total et du Ni(II), respectivement par analyse élémentaire et analyse polarographique, sur un prélèvement filtré au moyen d'un filtre Millipore Millex-HV ® (Hydrophilic PVDF 0.45 µm) fournit les résultats suivants : [Ni] = 129 mmol/kg et [Ni(II)] = 38 mmol/kg ([Ni°] = 91 mmol/kg).

### EXEMPLE 3

Dans un réacteur en verre de 150 cm³ purgé à l'argon et équipé d'une turbine auto-aspirante, on charge 35 cm³ de la suspension aqueuse évoquée dans l'exemple 1, § 2. Sous agitation (1200 tours/minute) et à une température de 70°C, on injecte en ciel de réacteur, avec un débit constant et sur une durée d'une heure, 0,265 ml d'acide cyanhydrique par l'intermédiaire d'une seringue thermostatée à -10°C. Après injection de l'HCN et retour à température ambiante, le mélange est maintenu sous agitation pendant 1 heure en système fermé, puis le ciel du réacteur est balayé par un flux d'argon pendant encore environ 1 heure. Par ce traitement, on obtient une solution aqueuse homogène : pas de précipité apparent au sein de la solution au repos. Un dosage du Ni total et du Ni(II), respectivement par analyse élémentaire et analyse polarographique, sur un prélèvement filtré au moyen d'un filtre Millipore Millex-HV ® (Hydrophilic PVDF 0.45 µm) fournit les résultats suivants : [Ni] = 122 mmol/kg et [Ni(II)] = 30 mmol/kg ([Ni°] = 92 mmol/kg).

### EXEMPLE 4

Dans un réacteur en verre de 150 cm³ purgé à l'argon et équipé d'une turbine auto-aspirante, on charge 35 cm³ de la suspension aqueuse évoquée dans l'exemple 1, § 2. Sous agitation (1200 tours/minute) et à une température de 50°C, on injecte en ciel de réacteur, avec un débit constant et sur une durée d'une heure, 0,295 ml d'acide cyanhydrique par l'intermédiaire d'une seringue thermostatée à -10°C. Après injection de l'HCN et retour à température ambiante, le mélange est maintenu sous agitation pendant 1 heure en système fermé, puis le ciel du réacteur est balayé par un flux d'argon pendant encore environ 1 heure. Par ce traitement, on obtient une solution aqueuse homogène : pas de précipité apparent au sein de la solution au repos. Un dosage du Ni total et du Ni(II), respectivement par analyse élémentaire et analyse polarographique, sur un prélèvement filtré au moyen d'un filtre Millipore Millex-HV ® (Hydrophilic PVDF 0.45 µm) fournit les résultats suivants : [Ni] = 132 mmol/kg et [Ni(II)] = 40 mmol/kg ([Ni°] = 92 mmol/kg).

### EXEMPLE 5

### 1) Séparation de la phase solide

La phase aqueuse récupérée après décantation est filtrée sous atmosphère d'argon sur un fritté de porosité 4. Le filtrat, conservé sous d'argon, se présente sous forme d'une solution homogène rouge foncé. Sur le fritté, on récupère une pâte verte qui, séchée sous vide à température ambiante durant 12h, conduit à une poudre de coloration vert clair. Le diagramme de diffraction des rayons X de cette dernière présente les raies de diffraction caractéristiques de Ni(OH)₂ cristallisé et du sel sodique de la TPPTS cristallisé. L'analyse élémentaire de la poudre en question fournit la composition suivante : Ni : 11,7% pds ; P : 3,7% pds ; C : 27,6% pds ; Na : 6,0% pds ; N : < 1% pds.

### 2) Traitement de la phase solide

Dans un réacteur en verre de 150 cm³ purgé à l'argon et équipé d'une turbine auto-aspirante, on charge 35 cm³ d'une solution de 24,6 mmol de sel de sodium de triphénylphosphine trisulfonée (TPPTS) dans l'eau. On dégaze cette solution. On introduit ensuite, 2,75 g de la poudre vert clair précédemment obtenue. Sous agitation (1200 tours/minute) et à température ambiante, on injecte en ciel de réacteur, avec un débit constant et sur une durée d'une heure, 0,540 ml d'acide cyanhydrique par l'intermédiaire d'une seringue thermostatée à -10°C. Au cours de l'injection de l'HCN, la solution devient rapidement orangée puis au rouge vif. Après injection, le mélange est maintenu sous agitation pendant 3 heures à 80°C, puis refroidi à température ambiante où il est maintenu encore 12 heures sous agitation. Après un balayage à l'argon du ciel du réacteur pendant environ1 heure, on récupère une solution homogène, limpide et de coloration rouge foncé. Un dosage du Ni sur un prélèvement filtré au moyen d'un filtre Millipore Millex-HV ® (Hydrophilic PVDF 0.45 µm) fournit le résultat suivant : [Ni] = 123 mmol/kg.

La solution ainsi récupérée peut être utilisée comme solution catalytique après avoir été soumise à une étape de régénération par réduction après addition de nickel à l'état d'oxydation (0).

## Revendications

1. Procédé d'hydrocyanation de composés organiques comprenant au moins une liaison éthylénique par réaction avec le cyanure d'hydrogène, en présence d'une solution aqueuse d'un catalyseur comprenant au moins un composé de nickel et au moins une phosphine hydrosoluble à au moins 0,01 g/l d'eau, et à séparer après réaction la phase organique de la phase aqueuse, **caractérisé en ce qu'**il consiste à traiter la phase aqueuse séparée comprenant une phase solide formée au cours de la réaction d'hydrocyanation par du cyanure d'hydrogène pour dissoudre au moins partiellement la phase solide.

2. Procédé selon la revendication 1, **caractérisé en ce que** la phase aqueuse séparée est soumise à une étape de séparation de la phase solide formée, le traitement par du cyanure d'hydrogène étant réalisé sur ladite phase solide séparée.

3. Procédé selon la revendication 1 ou 2, **caractérisé en ce que** la phase aqueuse séparée est recyclée partiellement, après dissolution de la phase solide formée, dans la réaction d'hydrocyanation de composés organiques éthyléniquement insaturé.

4. Procédé selon l'une des revendications 2 à 3, **caractérisée en ce que** la partie de la phase aqueuse séparée, non recyclée dans la réaction d'hydrocyanation est soumise à une étape de régénération du catalyseur.

5. Procédé selon la revendication 2 ou 3, **caractérisé en ce que** la solution aqueuse obtenue par traitement de la phase solide formée par du cyanure d'hydrogène est soumise à une étape de réduction au moins partielle du nickel à l'état (II), en du nickel à l'état d'oxydation (0).

6. Procédé selon la revendication 6, **caractérisé en ce que** la solution aqueuse obtenue par traitement de la phase solide formée par du cyanure d'hydrogène est mélangée, préalablement à l'étape de réduction avec au moins une partie de la phase aqueuse séparée.

7. Procédé selon l'une des revendications précédentes, **caractérisé en ce que** la réduction est réalisée par traitement par de l'hydrogène gazeux.

8. Procédé selon l'une des revendications 1 à 7, **caractérisé en ce que** la réduction est réalisée par un procédé de réduction chimique par un agent réducteur choisi dans le groupe comprenant les borohydrures, le magnésium, le zinc.

9. Procédé selon, l'une des revendications 1 à 7, **caractérisé en ce que** la réduction est réalisée selon un procédé électrochimique.

10. Procédé selon l'une des revendications précédentes **caractérisé en ce que** la réaction d'hydrocyanation comprend la réaction d'isomérisation des composés nitriles formés.

## Patentansprüche

1. Verfahren zur Hydrocyanierung von organischen Verbindungen, die mindestens eine ethylenisch ungesättigte Bindung umfassen, durch Reaktion mit Cyanwasserstoff in Anwesenheit einer wäßrigen Lösung eines Katalysators, der mindestens eine Verbindung von Nickel und mindestens ein in mindestens 0,01 g/l Wasser wasserlösliches Phosphin umfaßt, und Abtrennung der organischen Phase von der wäßrigen Phase nach der Reaktion, **dadurch gekennzeichnet, daß** es darin besteht, die abgetrennte wäßrige Phase, die eine im Verlauf der Reaktion der Hydrocyanierung durch den Cyanwasserstoff gebildete feste Phase umfaßt, zu behandeln, um mindestens teilweise die feste Phase aufzulösen.

2. Verfahren nach Anspruch 1, **dadurch gekennzeichnet, daß** die abgetrennte wäßrige Phase einer Stufe zur Abtrennung der gebildeten festen Phase unterzogen wird, wobei die Behandlung durch Cyanwasserstoff an der genannten abgetrennten festen Phase realisiert wird.

3. Verfahren nach Anspruch 1 oder 2, **dadurch gekennzeichnet, daß** die abgetrennte wäßrige Phase nach der Auflösung der gebildeten festen Phase in die Reaktion der Hydrocyanierung von organischen, ethylenisch ungesättigten Verbindungen partiell zurückgeführt wird.

4. Verfahren nach einem der Ansprüche 2 bis 3, **dadurch gekennzeichnet, daß** der Teil der abgetrennten wäßrigen Phase, der nicht in die Reaktion der Hydrocyanierung zurückgeführt wird, einer Stufe zur Regenerierung des Katalysators unterzogen wird.

5. Verfahren nach Anspruch 2 oder 3, **dadurch gekennzeichnet, daß** die wäßrige Lösung, die durch Behandlung der gebildeten festen Phase durch Cyanwasserstoff erhalten wird, einer Stufe zur mindestens partiellen Reduktion des Nickels vom Zustand (II) zum Nickel vom Oxidationszustand (0) unterzogen wird.

6. Verfahren nach Anspruch 5, **dadurch gekennzeichnet, daß** die wäßrige Lösung, die durch Behandlung der gebildeten festen Phase durch Cyanwasserstoff erhalten wird, vor der Stufe der Reduktion mit mindestens einem Teil der abgetrennten wäßrigen Phase vermischt wird.

7. Verfahren nach einem der vorstehenden Ansprüche, **dadurch gekennzeichnet, daß** die Reduktion durch Behandlung mit gasförmigem Wasserstoff realisiert wird.

8. Verfahren nach einem der Ansprüche 1 bis 7, **dadurch gekennzeichnet, daß** die Reduktion durch ein Verfahren zur chemischen Reduktion mit einem Reduktionsmittel realisiert wird, das aus der die Borhydride, Magnesium, Zink umfassenden Gruppe gewählt wird.

9. Verfahren nach einem der Ansprüche 1 bis 7, **dadurch gekennzeichnet, daß** die Reduktion nach einem elektrochemischen Verfahren realisiert wird.

10. Verfahren nach einem der vorstehenden Ansprüche, **dadurch gekennzeichnet, daß** die Reaktion der Hydrocyanierung die Reaktion der Isomerisierung der gebildeten Nitrilverbindungen umfaßt.

## Claims

1. Process for the hydrocyanation of organic compounds comprising at least one ethylenic bond by reaction with hydrogen cyanide, in the presence of an aqueous solution of a catalyst comprising at least one nickel compound and at least one phosphine which is soluble in water to at least 0.01 g/l of water, and by separation, after reaction, of the organic phase from the aqueous phase, **characterized in that** it consists in treating the separated aqueous phase, comprising a solid phase formed during the hydrocyanation reaction, with hydrogen cyanide in order to at least partially dissolve the solid phase.

2. Process according to Claim 1, **characterized in that** the separated aqueous phase is subjected to a stage of separation of the solid phase formed, the treatment with hydrogen cyanide being carried out on the said separated solid phase.

3. Process according to Claim 1 or 2, **characterized in that** the separated aqueous phase is partially recycled, after dissolution of the solid phase formed, in the reaction for the hydrocyanation of ethylenically unsaturated organic compounds.

4. Process according to either of Claims 2 and 3, **characterized in that** the part of the separated aqueous phase not recycled in the hydrocyanation reaction is subjected to a stage of regeneration of the catalyst.

5. Process according to Claim 2 or 3, **characterized in that** the aqueous solution obtained by treatment with hydrogen cyanide of the solid phase formed is subjected to a stage of at least partial reduction of nickel in the (II) state to nickel in the (0) oxidation state.

6. Process according to Claim 5, **characterized in that** the aqueous solution obtained by treatment with hydrogen cyanide of the solid phase formed is mixed, prior to the reduction stage, with at least a portion of the separated aqueous phase.

7. Process according to one of the preceding claims, **characterized in that** the reduction is carried out by treatment with gaseous hydrogen.

8. Process according to one of Claims 1 to 7, **characterized in that** the reduction is carried out by a process of chemical reduction by a reducing agent chosen from the group consisting of borohydrides, magnesium and zinc.

9. Process according to one of Claims 1 to 7, **characterized in that** the reduction is carried out according to an electrochemical process.

10. Process according to one of the preceding claims, **characterized in that** the hydrocyanation reaction comprises the isomerization reaction of the nitrile compounds formed.
